# EUROPÄISCHE PATENTSCHRIFT

(11) **EP 1 745 812 B1**
(45) Veröffentlichungstag und Bekanntmachung des Hinweises auf die Patenterteilung: **03.12.2008**
(21) Anmeldenummer: 05015966.4
(22) Anmeldetag: 22.07.2005
(51) Int. Cl.: A61M 5/152, G05D 16/06

(54) **Ventil für ein Fluid, insbesondere zur Verwendung in einer mechanisch betriebenen Flüssigkeitspumpe**
Valve for a fluid, in particular for being used in a mechanically actuated liquid pump
Soupape pour un fluide, en particulier pour être utilisée dans une pompe à liquide actionnée méchaniquement

(43) Veröffentlichungstag der Anmeldung: 24.01.2007
(73) Patentinhaber: RoweMed AG - Medical 4 Life, 19370 Parchim (DE)
(72) Erfinder: Wex, Roland, 34212 Melsungen (DE); Hansmann, Harald, Prof. Dr,-Ing., 23966 Wismar (DE); Kenzler, Christian, Dipl.-Ing, 16928 Pritzwalk (DE)
(74) Vertreter: Quermann, Helmut

(56) Entgegenhaltungen:
- EP-A- 0 475 743
- EP-A- 0 812 596
- EP-A- 1 321 156
- US-A- 3 386 469
- US-A1- 2004 168 723

## Beschreibung

Die Erfindung betrifft ein Ventil für ein Fluid, insbesondere für eine Flüssigkeit, wobei das Ventil durch das zu regelnde Fluid betätigt wird, mit einem Gehäuse, wobei das Ventil eine im Gehäuse gelagerte elastische Ventilmembran und einen in dieser gehaltenen Ventilkörper aufweist, sowie mit einem Zugang für das Fluid, der in eine Kammer mündet und mit einem von der Kammer abgehenden Abgang, wobei der Ventilkörper zum Schließen des Fluiddurchgangs des Ventils in Kontakt mit einem elastischen Element bringbar ist.

Das Ventil findet vorzugsweise bei einer mechanisch betriebenen Flüssigkeitspumpe Verwendung, insbesondere bei einer solchen Flüssigkeitspumpe, die für medizinische und ernährungsphysiologische Flüssigkeiten, sowie Flüssigkeiten in biologischen und Laborbereich vorgesehen ist. Es ist genauso denkbar, das Ventil für Gase zu verwenden, insbesondere solche, die im Rahmen von Laborversuchen zugeführt werden müssen. Das Haupteinsatzgebiet des Ventils liegt auf dem Gebiet des Förderns einer Flüssigkeit.

Im Zusammenhang mit der Förderung von Flüssigkeiten werden die unterschiedlichsten Typen von Flüssigkeitspumpen verwendet. Nicht elektrisch betriebene Pumpen, vorzugsweise mechanische Infusionspumpen, haben den Nachteil, dass sie innerhalb der Einsatzzeit unterschiedliche Drücke erzeugen. Dies bedeutet, dass der Anfangsdruck in der Regel höher als der Enddruck ist. Die Flowratenregelung wird über Kapillarschläuche oder Glaskapillarrohre vorgenommen. Diese Kapillarlösungen ergeben innerhalb der Anwendungszeit Flowratenschwankungen von bis zu 70 %. Dies ist für Patienten mit Toleranzproblemen, z.B. Allergiker, ein großes Problem, da zu hohe Dosierraten zu Nebenwirkungen und Unverträglichkeiten führen, bis hin zur Todesfolge.

Ein Ventil der eingangs genannten Art, das für Flüssigkeiten Verwendung findet und welches alle Merkmale des Oberbegriffs von Anspruch 1 zeigt, ist aus der EP 1 321 156 A1 bekannt. Dort weist das Gehäuse eine Kammer, einen in Strömungsverbindung mit der Kammer stehenden Eintrittskanal und einen in Strömungsverbindung mit der Kammer stehenden Austrittskanal für die Flüssigkeit auf. Innerhalb der Kammer ist ein steifer Ventilkörper zum Regeln des Durchflusses der Flüssigkeit durch die Kammer angeordnet. Der Ventilkörper ist mit einer im Bereich des umlaufenden Randes im Gehäuse geklemmten Ventilmembran verbunden, womit der Ventilkörper entsprechend der Bewegung der Ventilmembran bewegt wird. Konzentrisch zum Eintrittskanal nimmt das Gehäuse ein ringförmiges elastisches Element auf. Zum Verschließen des Eintrittskanals ist der Ventilkörper gegen das elastische Element bewegbar. Aufgrund der Dimensionierung dieses Ventils und des dem Austrittskanal zugeordneten Durchflussbegrenzers ist der Widerstand, der der Flüssigkeit beim Ausströmen aus dem Gehäuse entgegengesetzt wird, größer als der Widerstand, der der in das Ventil eintretenden Flüssigkeit entgegengesetzt wird. Beim Einströmen der Flüssigkeit in das Gehäuse entsteht in der mit Flüssigkeit gefüllten Kammer ein hydrostatischer Druck, der auf den Ventilkörper und die Ventilmembran wirkt. Bei drucklosem Anliegen von Distanzansätzen des Ventilkörpers am elastischen Element wird der dieses durchsetzende Flüssigkeitskanal nicht verschlossen und es kann Flüssigkeit aus dem Eintrittskanal in die Kammer und von dort in den Austrittskanal strömen. Bedingt durch die Elastizität des elastischen Elements wird durch eine in Richtung auf dieses wirkende Kraft, bei Druckänderung der in der Kammer befindlichen Flüssigkeit, die Ventilmembran geringfügig vom Kammerinneren weg gewölbt und der Ventilkörper in dieser Richtung bewegt, wobei die Distanzansätze des Ventilkörpers in das elastische Element eingedrückt werden. Hierdurch reduziert sich der Spalt zwischen den Dichtflächen. Der durch das Ventil durchtretende Volumenstrom wird damit geringer. Bei ausreichend großer Kraft schließt der Ventilkörper den Spalt, so dass im Eintrittskanal befindliche Flüssigkeit nicht mehr in die Kammer eindringen kann. Hierdurch bedingt baut sich in der Kammer der Druck ab. Bei Druckabbau führt die Rückstellkraft der ausgelenkten Ventilmembran dazu, dass der Ventilkörper vom elastischen Element abgehoben wird, womit sich wieder der Spalt zwischen den Dichtflächen bildet. Dieses Ventil arbeitet somit in Art eines Druckreduzierers, und zwar in Abhängigkeit vom anstehenden Eingangsdruck. Es ist mit diesem Ventil nur unzureichend möglich, den Druck weitgehend konstant zu regeln.

In der US 5,616,127 ist eine Vorrichtung zum Zuführen einer medizinischen Flüssigkeit zu einem Patienten beschrieben. Diese weist ein Ventil zum Regeln des Durchflusses des flüssigen Medikaments auf. Das Ventil weist ein Gehäuse mit konisch angeordnetem Ventilsitz und einen relativ zu diesem verschieblichen Ventilkörper mit gleichfalls konischem Ventilsitz auf. Der Ventilkörper ist über ein elastisches Zwischenglied in Art eines Balges mit dem Ventilgehäuse verbunden. Der vom Balg umschlossene Raum bildet eine Kammer, die mit einem Auslasskanal versehen ist. Der Einlasskanal ist durch das Ventilgehäuse geführt. Bei einem Anstieg des Drucks in der Kammer weitet sich der Balg, womit der Ventilkörper den Durchtrittsquerschnitt des Ventilgehäuses reduziert, bis bei einem Maximaldruck der Einlasskanal des Ventilgehäuses mittels des Ventilkörpers verschlossen wird. Ist das Ventil drucklos, sorgt der Balg aufgrund dessen Rückstellkräften dazu, dass der Ventilkörper gegen einen weiteren Ventilsitz des Gehäuses bewegt wird und auch in diesem Zustand den Durchfluss durch das Ventil sperrt.

Aus der US 4,852,605 ist ein Ventil bekannt, bei dem ein Ventilgehäuse einen Ventilsitz aufweist, mit dem ein Ventilkörper zusammenwirkt, der als Kugel ausgebildet ist. Diese Kugel ist in einem Lagerkörper gehalten, der seinerseits in einer Ventilmembran gelagert ist. Bei Anliegen eines erhöhten Drucks in einem Einlasskanal des Gehäuses hebt die Kugel vom Ventilsitz ab. In Abhängigkeit von der Druckhöhe ergibt sich ein mehr oder minder großer Austrittsquerschnitt für die Flüssigkeit aus dem Einlasskanal.

In der DE 44 36 540 A1 ist ein Infusionssystem zur kontinuierlichen Abgabe eines flüssigen Medikaments unter Druck beschrieben. Dort befindet sich ein Kolben in einem Gleichgewichtszustand. Wird über eine Medikamentenabgabevorrichtung ein zu höher Volumenstrom appliziert, führt dies zu einer Verschiebung des Kolbens und, in Abhängigkeit vom Verschiebeweg des Kolbens zu einer mehr oder minder großen Abblendung eines Einlasskanals für das Medikament.

In der US 3,511,472 ist ein Ventil mit elastischer Ventilmembran zum Verschließen des Sitzes eines Ventilgehäuses beschrieben. Der Durchtrittsquerschnitt des Ventils lässt sich durch eine den Ventilkörper tragende, verstellbare Schraube stufenlos regulieren.

Es ist Aufgabe der vorliegenden Erfindung, ein Ventil der eingangs genannten Art so weiter zu bilden, dass ein weitgehendes Konstanthalten des Flüssigkeitsstromes, somit ein Konstanthalten des Flüssigkeitsstromes in einem engen Toleranzbereich, gewährleistet ist.

Gelöst wird die Aufgabe durch ein Ventil gemäß Anspruch 1, d.h. der eingangs genannten Art, das gekennzeichnet ist durch folgende Merkmale:
- das elastische Element ist die elastische Ventilmembran,
- ein sich außerhalb des Fluidweges befindlicher Anschlag dient dem Begrenzen der Auswölbbewegung der Ventilmembran bei einem Druckanstieg des Fluids, wobei der Anschlag das elastische Element gegen den Ventilkörper bewegt und den Fluiddurchgang des Ventils schließt,
- der Zugang ist durch die Ventilmembran und den Ventilkörper hindurch zur Kammer geführt,
- der am Ventilabgang anstehende Druck führt über eine entsprechend groß gestaltete Wirkfläche zu einer Auswölbung der Ventilfläche.

Von besonderem Vorteil ist bei dem erfindungsgemäßen Ventil, dass der Flüssigkeitsstrom, unabhängig davon, wie groß der anstehenden Zugangsdruck am Ventil ist, im wesentlichen konstant geregelt werden kann. Dies ist deshalb möglich, weil ein am Abgang anstehender Druck über eine entsprechend groß gestaltete Wirkfläche zu einer Auswölbung der Ventilmembran aus deren Ausgangslage führt, mit der Konsequenz, dass die Ventilmembran gegen den Anschlag bewegt wird und hierdurch eine Verformung erfährt, die zum Verschluss des Ventilkörpers führt. Der relativ hohe Druck im Bereich des Abgangs, der selbstverständlich niedriger ist als der Druck im Bereich des Zugangs, wird durch den Durchflussbegrenzer sichergestellt.

Besonders vorteilhaft ist dabei, das erfindungsgemäß der Eingang des Ventils durch die Ventilmembran und den Ventilkörper hindurch zur Kammer geführt ist. Es handelt sich insbesondere um miteinander verbundene Kanäle.

Die Ventilmembran und der Ventilkörper sind insbesondere separate Bauteile. Der Ventilkörper ist insbesondere als Ventilkern ausgebildet. Durch diese Formulierung soll zum Ausdruck kommen, dass dieses Bauteil in einem zentralen Bereich des Ventils angeordnet ist, insbesondere in der beschriebenen Weise in der Ventilmembran gehalten ist. Der Ventilkörper ist vorzugsweise ein starres Bauteil. Er ist derart in der Ventilmembran gehalten, dass er weder in der Bewegungsrichtung des Ventilkörpers relativ zu diesem bewegt werden kann, noch in dessen Querrichtung, insbesondere auch nicht bezüglich der Ventilmembran, verdreht werden kann. Hierdurch ist sichergestellt, dass in jedem Betriebszustand die Zugangskanäle von Ventilmembran und Ventilkörper fluchten und überdies eine definierte Position des Ventilkörpers zur Ventilmembran, insbesondere im Bereich in dem der Ventilkörper durch die Ventilmembran verschlossen werden soll, gewährleistet ist. Zweckmäßig ist die Ventilmembran im Bereich eines umlaufenden Randes zwischen zwei Teilen des Gehäuses gehalten, insbesondere klemmend gehalten. Hierbei besteht die Möglichkeit, ausgehend von den Randbereichen der Ventilmembran, diese ausreichend unter dem Einfluss des jeweils anstehenden Fluiddrucks zu wölben. Die Ventilmembran ist vorzugsweise im Querschnitt profiliert ausgebildet und es dient ein zentraler Bereich der Ventilmembran der Aufnahme des Ventilkörpers. Dieser zentrale Bereich ist insbesondere topfförmig ausgebildet.

Zweckmäßig ist eine besondere Gestaltung der Ventilmembran vorgesehen. Demgemäß ist ein Verschlusselement Bestandteil der Ventilmembran. Der Anschlag bewegt das Verschlusselement gegen den Ventilkörper und schließt dabei den Fluidabgang des Ventils.

Der Abgang des Ventils kann auf unterschiedliche Art und Weise ausgeführt sein. Es ist denkbar, den Abgang unmittelbar von der Kammer zum Gehäuse zu führen und von dort über einen Kanal zu einer mit dem Gehäuse verbundenen Abgangsleitung. Eine modifizierte Gestaltung sieht vor, dass, entsprechend der Führung des Zugangskanals durch die Ventilmembran, durchaus auch ein Abgangkanal vorgesehen sein kann, der durch die Ventilmembran hindurchgeführt ist.

Konstruktiv lässt sich das Ventil im Bereich der Ventilmembran und des Ventilkörpers besonders einfach gestalten, wenn die Ventilmembran einen radialen Einlasskanal und der Ventilkörper einen sich an diesen Einlasskanal anschließenden radialen Einlasskanal aufweist, wobei letzterer in einen axialen Einlasskanal des Ventilkörpers mündet, der mittels der Ventilmembran verschließbar ist. Zwischen dem Ventilkörper und der Ventilmembran ist zweckmäßig, von der Kammer ausgehend, ein Auslasskanal gebildet.

Gemäß einer besondere Ausführungsform der Erfindung ist vorgesehen, dass der Anschlag als verstellbarer Anschlag gestaltet ist. Dieser ist insbesondere als in das Gehäuse eingeschraubte Stellschraube ausgebildet. Je weiter die Schraube den Stellweg der Ventilmembran freigibt, desto größer ist der Sekundärdruck im Ventil.

Mit dem erfindungsgemäßen Ventil erfolgt die Regelung des Durchflusses des Fluids bezüglich des Maximaldrucks. Die Ventilmembran ist hierbei vorzugsweise auf das zu regelnde Druckniveau ausgelegt. Dies bedeutet, dass die Regelung durchaus mittels der elastischen Ventilmembran erfolgen kann. - Gemäß einer Weiterbildung der Erfindung ist allerdings eine Feder zum Rückstellen der Ventilmembran in die Offenstellung des Ventils vorgesehen. Diese unterstützt die Rückstellbewegung der Ventilmembran.

Die Feder ist vorzugsweise als Druckfeder ausgebildet, die sich am Gehäuse und der Ventilmembran abstützt, wobei insbesondere die Ventilmembran gegen einen gehäuseseitigen Anschlag rückstellbar ist. Dieser Anschlag begrenzt die Rückstellbewegung der Ventilmembran.

Der Auslass des Ventils weist vorzugsweise einen Durchflussbegrenzer auf. Dieser bildet damit Bestandteil des Ventils.

Weitere Merkmale der Erfindung sind in den Unteransprüchen, der Beschreibung der Figuren sowie den Figuren selbst dargestellt.

In den Figuren ist die Erfindung anhand einer mechanisch betriebenen Flüssigkeitspumpe, die mit dem Ventil versehen ist, veranschaulicht, ohne auf dieses Ausführungsbeispiel beschränkt zu sein. Es zeigt:
- Figur 1: eine Explosionsdarstellung einer mechanisch betriebenen Flüssigkeitspumpe unter Verwendung des erfindungsgemäßen Ventils,
- Figur 2: einen vertikalen Längsmittelschnitt durch die in Figur 1 gezeigte Pumpe, , insbesondere zur Veranschaulichung des Antriebs der Pumpe, mit einem an einem Kern anliegenden Ballon,
- Figur 3: ein Schnitt gemäß Figur 2, bei mit Flüssigkeit gefülltem Ballon,
- Figur 4: einen vertikalen Längsschnitt durch die in Figur 1 gezeigte Pumpe, in Abstand zur Längsmittelachse der Pumpe, im Bereich des Ventils der Pumpe geschnitten,
- Figur 5: einen Schnitt, quer durch die in Figur 1 veranschaulichte Pumpe geschnitten, im Bereich des Ventils,
- Figur 6: einen horizontalen Längsmittelschnitt durch die in Figur 1 gezeigte Pumpe, bei am Kern anliegenden Ballon,
- Figur 7: einen Schnitt gemäß Figur 6, bei mit Flüssigkeit gefülltem Ballon,
- Figur 8: einen Schnitt durch die Pumpe, quer geschnitten im Bereich der Lagerung des Kernes,
- Figur 9: einen Schnitt durch die Pumpe, quer geschnitten im Bereich des nicht gelagerten Abschnitts des Kernes und des mit Flüssigkeit gefüllten Ballons,
- Figur 10: eine vergrößerte Schnittdarstellung von Kern, Ballon und Klemmring zum Verbinden von Ballon und Kern, bei am Kern anliegenden Ballon,
- Figur 11: einen Schnitt, quer zur Längserstreckung des Kerns geschnitten, durch den Kern und den Ballon, bei am Kern anliegenden Ballon,
- Figur 12: eine Schnittdarstellung gemäß Figur 11 für eine modifizierte Querschnittsgestaltung des Ballons,
- Figur 13: eine vergrößerte Schnittdarstellung des in Figur 4 gezeigten Ventils, und
- Figur 14: ein Schaubild zur Verdeutlichung des Wirkprinzips der mechanisch betriebenen Flüssigkeitspumpe mit Angabe physikalischer Größen.

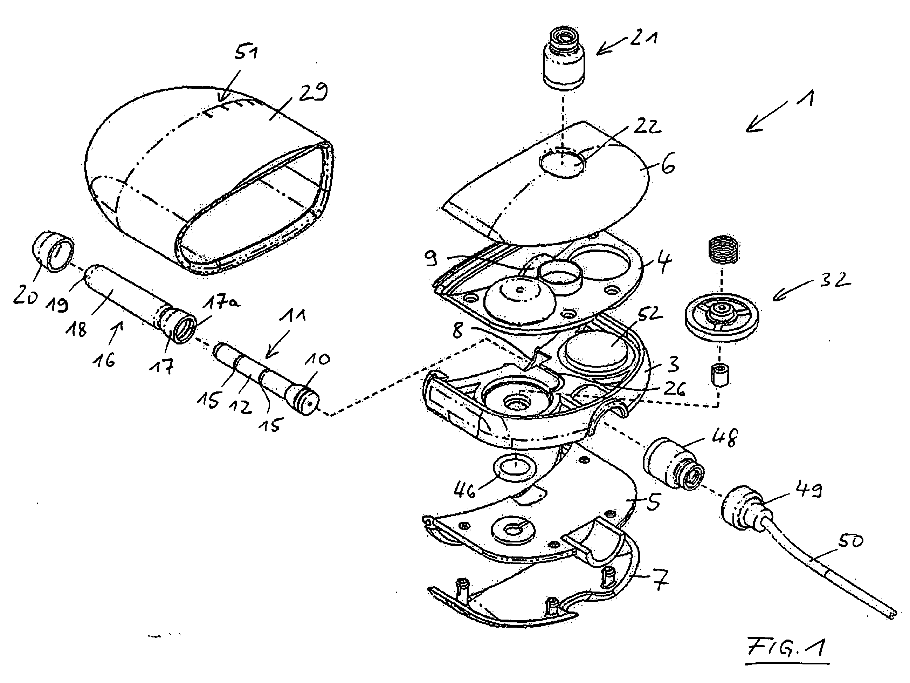

Die in Figur 1 veranschaulichte mechanisch betriebene Flüssigkeitspumpe 1 dient insbesondere der Applikation von medizinischen oder ernährungsphysiologischen Flüssigkeiten, beispielsweise der Applikation eines flüssigen Medikaments.

Die Pumpe 1 weist ein mehrteiliges Gehäuse 2 auf, das durch ein Mittelteil 3, mit diesem zusammenwirkenden Oberteil 4 und Unterteil 5, eine mit dem Oberteil zusammenwirkende Oberschale 6 und eine mit dem Unterteil 5 zusammenwirkende Unterschale 7 gebildet ist.

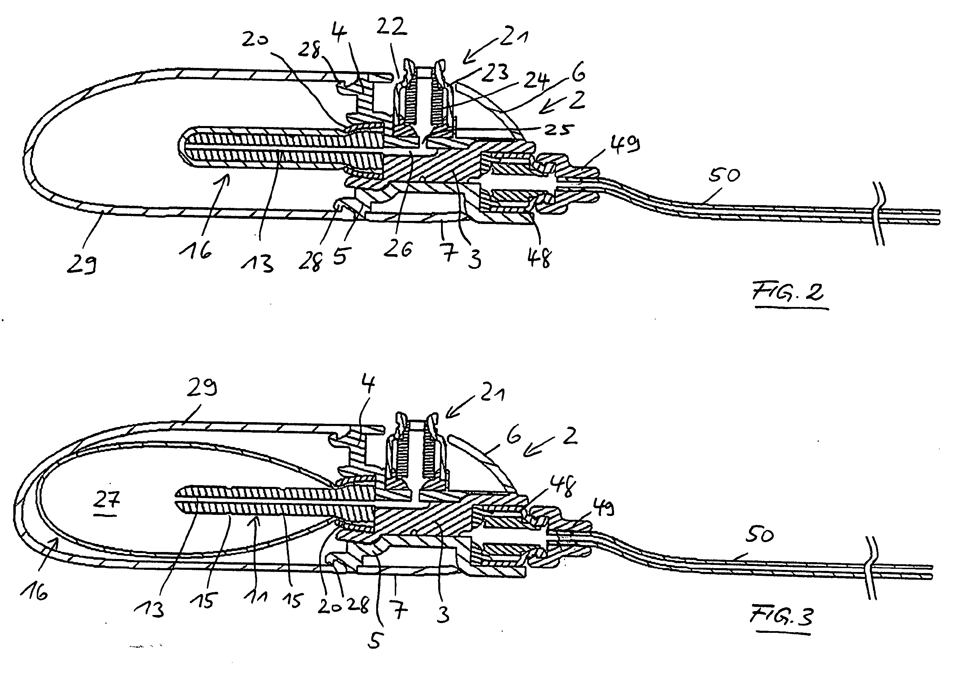

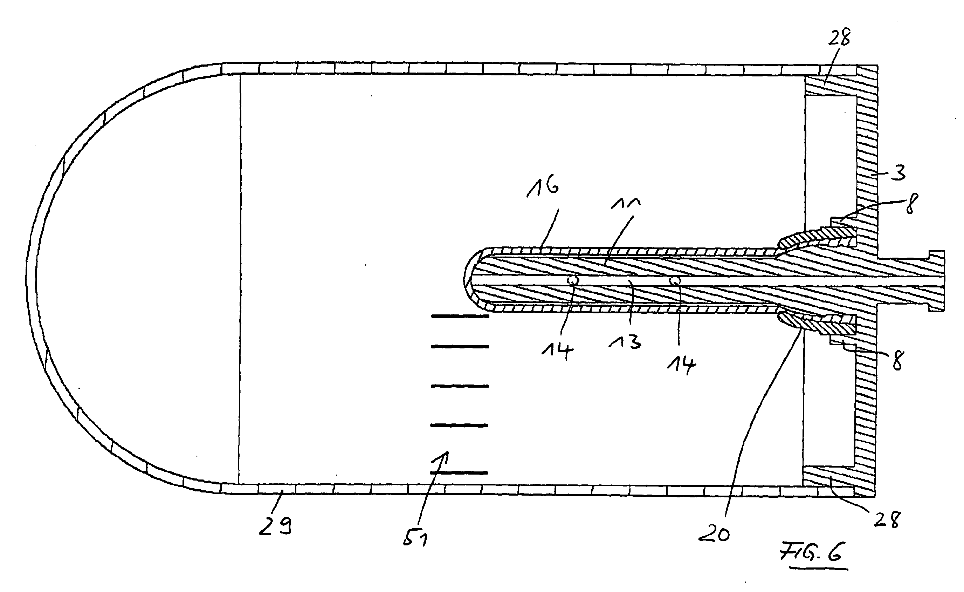

Das Mittelteil 3 ist auf seiner Oberseite mit einer zum freien Rand des Mittelteils 3 offenen, im Querschnitt halbkreisförmigen Ausnehmung 8 und das Oberteil 4 im entsprechenden Randbereich auf seiner Unterseite mit einer entsprechenden halbkreisförmigen Ausnehmung 9 versehen. Bei mit dem Mittelteil 3 verbundenem Oberteil 4 bilden die beiden Ausnehmungen 8 und 9 einen kreisförmigen Querschnitt zur Aufnahme eines konusförmig erweiterten Endbereichs 10 eines Kernes 11. Dieser weist, abgesehen von dessen Endbereich 10, einen konstanten Außendurchmesser auf. Dieser zylindrische Abschnitt des Kerns 11 ist mit der Bezugsziffer 12 bezeichnet. Den Kern 11 durchsetzt in dessen Längsmittelachse ein Kanal 13 (siehe Figur 2 ) von dem mehrere sich radial durch den Kern 11 erstreckende Kanäle 14 im Bereich des Abschnitts 12 abzweigen (Figur 6). Im Bereich des äußeren Umfangs des Kerns 10 münden die radialen Kanäle 14 in umlaufende Nuten 15 des Kerns 11.

Mit dem Kern 11 wirkt ein elastisches Element zusammen, dass als aus Silikon bestehender Ballon 16 ausgebildet ist. Dieser ist im Spritzgussverfahren hergestellt. Der Ballon weist entsprechend dem Endbereich 10 des Kerns 11 einen sich konisch erweiterten Endbereich 17 mit Öffnung 17a und einen der äußeren Form des Abschnitts 12 des Kerns 11 entsprechenden Abschnitt 18 auf, der in den, Ballon bedingt, geschlossenen Endbereich 19 mündet, der dem Endbereich 17 abgewandt ist.

Die Abmessungen von Kern 11 und Ballon 16 sind so bemessen, dass, wie es der Figur 2 zu entnehmen ist, der auf den Kern 11 aufgesteckte Ballon vollständig am Kern 11 anliegt, somit der Endbereich 17 des Ballons den Endbereich 10 des Kerns kontaktiert und der Abschnitt 18 des Ballons 16 den Abschnitt 12 des Kerns 11 kontaktiert, schließlich der Endbereich 19 des Ballons 16 am stirnseitigen freien Ende des Kerns 11 anliegt. Die Abmessungen des Ballons 16 bezüglich des Kerns 11 sind hierbei so gewählt, dass der Ballon 16 mit relativ geringer Vorspannung, somit in relativ entspanntem Zustand am Kern 11 anliegt.

Zum Befestigen des Ballons 16 im Bereich seines Endbereichs 17 am Kern 11 im Bereich dessen Endbereichs 10 ist ein Klemmring 20 vorgesehen, der außen auf den Ballon 16 im Bereich dessen Endbereichs 17 aufgesteckt wird. Das so geschaffene Gebilde wird mit dem Klemmring 20 in die Ausnehmung 8 des Mittelteils 3 eingelegt, anschließend das Oberteil 4 mit dem Mittelteil 3 verbunden, womit der Klemmring 20 und damit der Kern 11 sowie der Ballon 16 fest in den Ausnehmungen 8 und 9 von Mittelteil 3 und Oberteil 4 gehalten sind. Die Ausnehmungen 8 und 9 weisen eine sich vom jeweiligen freien Rand des Mittelteils 3 bzw. Oberteil 4 weg erweiternde konische Aufnahme für den Klemmring 20 auf, um einen sicheren Halt des Klemmrings 20 zu gewährleisten.

Das Mittelteil 3, das Oberteil 4 und das Unterteil 5 dienen der Aufnahme weiterer Funktionselemente der Pumpe 1:

Mit dem Oberteil 4 ist ein LuerLock-Ventil 21 verbunden, dass eine Öffnung 22 im Oberteil 4 durchsetzt und, wie es der nachfolgend Bezug genommenen Beschreibung der Figur 2 zu entnehmen ist, ein LuerLock-Ventilgehäuse 23 und einen LuerLock-Ventilkern 24 besitzt. Über einen Kanal 25 steht das LuerLock-Ventil 21 mit einem Kanal 26 in Verbindung, der zwischen dem Oberteil 4 und dem Mittelteil 3 gebildet ist und der mit dem den Kern 11 durchsetzenden Kanal 13 in Verbindung steht.

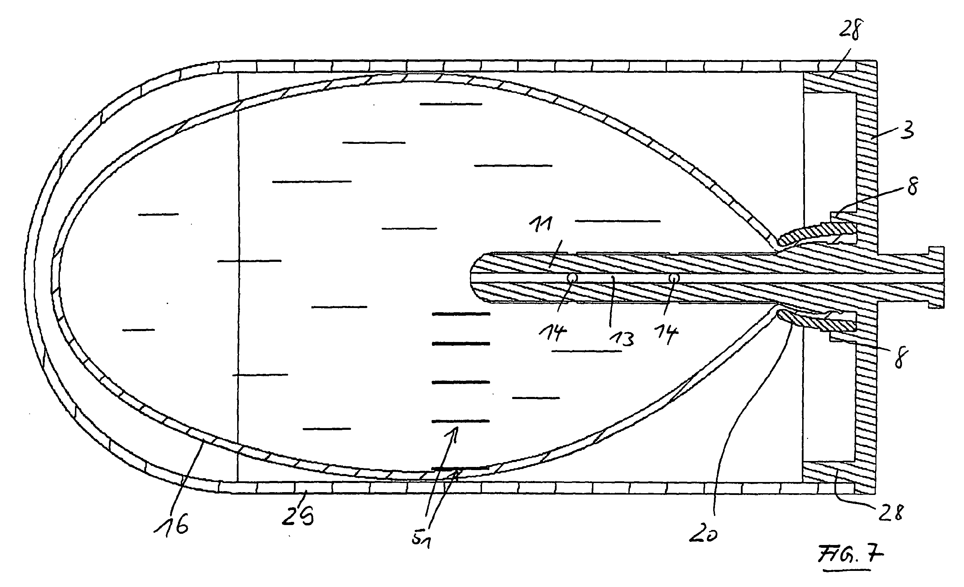

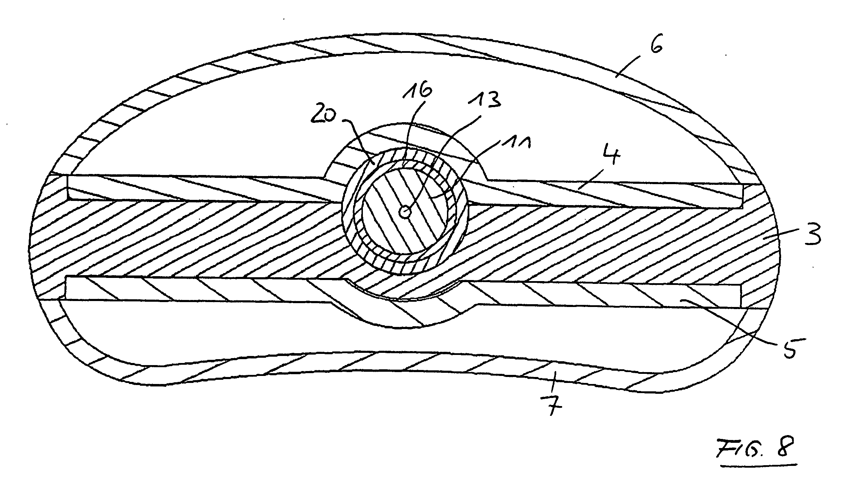

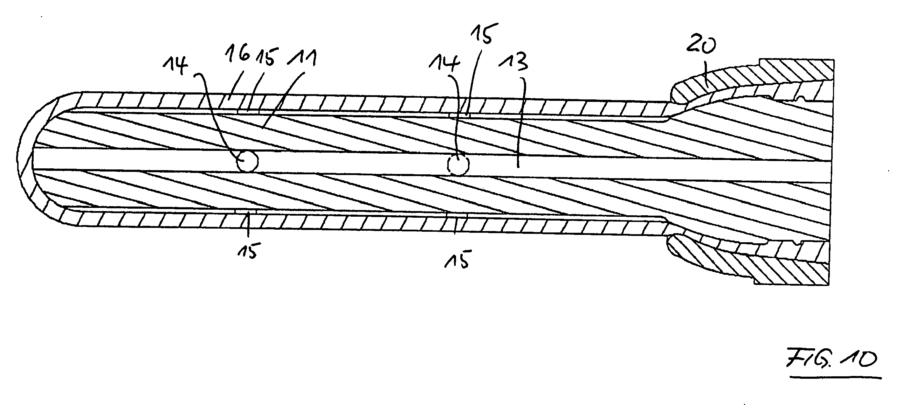

Durch das LuerLock-Ventil 31 und die Kanäle 25, 26 und 13 wird die Pumpe mit Flüssigkeit befüllt. Ausgehend von dem in Figur 2 veranschaulichten unbefüllten Zustand dehnt sich mit zunehmender Zugabe von Flüssigkeit der Ballon 16 in demjenigen Bereich, der nicht durch den Klemmring 20 geklemmt ist und nimmt die in Figur 3 veranschaulichte Endform bei vollständiger Füllung an. Der Raum, der von der Flüssigkeit eingenommen wird, ist dort mit der Bezugsziffer 27 bezeichnet. Den Figuren 2, 3 und 6 bis 12 ist zu entnehmen, dass sich der Ballon 16, ausgehend von seinem am Kern 11 anliegenden Ausgangszustand beim Befüllen mit Flüssigkeit seine Form sowohl in Längsrichtung des Kerns als auch in dessen Querrichtungen verändert, d. h. in einer ersten Querrichtung und einer senkrechten hierzu gesehen zweiten Querrichtung.

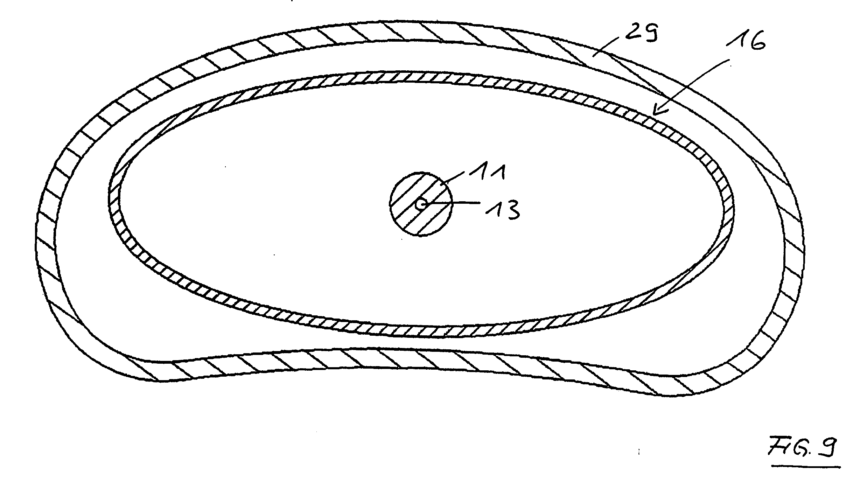

Das Oberteil 4 und das Unterteil 5 sind mit Rastvorsprüngen 28 versehen, die der Aufnahme einer im Querschnitt annähernd nierenförmig gestalteten Haube 29 dienen. Diese Haube besitzt, wie es anschaulich der Figur 9 zu entnehmen ist, eine Erstreckung in Breitenrichtung, die wesentlich größer ist als die in Höhenrichtung. Das Breiten-/Höhenverhältnis beträgt beispielsweise 2:1. Das Längen-/Höhenverhältnis der Haube 29 ist, wie es beispielsweise der Figur 2 zu entnehmen ist, ungefähr 2,5:1. Die Haube 29 ist vorzugsweise unlösbar mit dem Gehäuse 2 verclipst. Bei vollständig mit Flüssigkeit befülltem Ballon 16 nimmt dieser soviel wie möglich von dem Innenraum der Haube 29 ein.

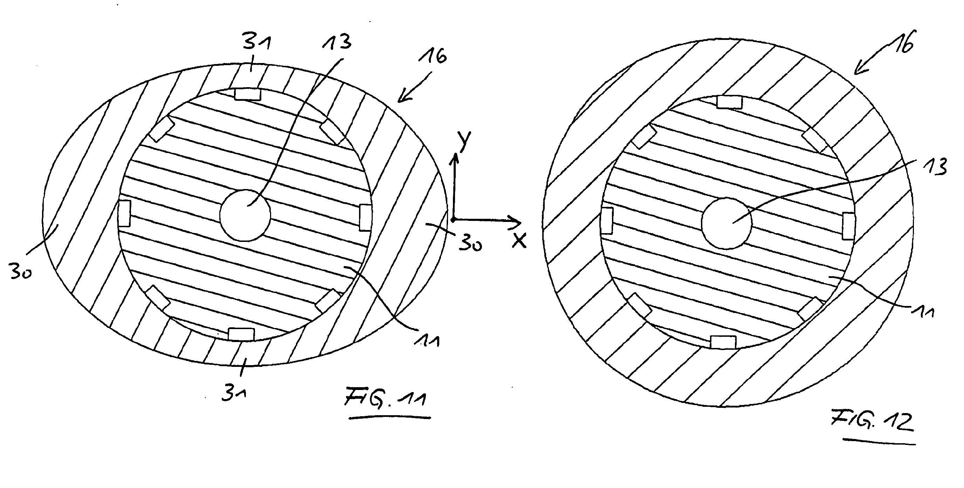

Erreicht wird dies dadurch, dass, wie es der Darstellung der Figur 11 für den am Kern 11 anliegenden Ballon 16 zu entnehmen ist, der Ballon 16 in einer ersten Erstreckungsrichtung X senkrecht zur Längsachse des Kerns 11 relativ dicke Wandungsabschnitte 30 und in einer zweiten Erstreckungsrichtung Y senkrecht zur Längsachse des Kerns 11 und senkrecht zur ersten Erstreckungsrichtung X relativ dünne Wandungsabschnitte 31 aufweist. Demzufolge hat der Ballon 16 beim Einbringen von Flüssigkeit in dessen Raum 27 das Bestreben sich bevorzugt in Erstreckungsrichtung X auszudehnen, womit die ausgedehnte ovale Querschnittsform, wie sie in der Darstellung der Figur 9 veranschaulicht ist, sich ergibt. Insgesamt stellt sich die Pumpe 1 als flaches, günstig am Körper zu tragendes Funktionsteil dar, wobei der Ballon 16 in mit Flüssigkeit gefülltem Zustand gleichfalls eine flache Form einnimmt, die der äußeren Kontur der Pumpe 1 angepasst ist.

Die Kanäle 26 und 13 dienen nicht nur dem Zuführen der Flüssigkeit vom Luer-Lock-Ventil 21 in den Ballon 16, sondern auch dem Abführen der Flüssigkeit aus dem Inneren des Ballons 16 zum Patienten. So ist der Kanal 26 über die Zugangsstelle des Kanals 25 hinaus verlängert zu einem im Mittelteil 3 und im Oberteil 4 gelagerten Ventil 32 zum Begrenzen des von dem Ballon 16 ausgegebenen Flüssigkeits-Volumenstroms. Dieses Ventil 32 ist durch eine randseitig zwischen Mittelteil 3 und Oberteil 4 gehaltene elastische Ventilmembran 33, einen mit dieser zusammenwirkenden Ventilkern 34, eine sich an der Ventilmembran 33 und dem Oberteil 4 abstützende Druckfeder 35 und eine in einem Gewinde des Oberteils 4 gelagerte Stellschraube 36, die in Wirkverbindung mit der Ventilmembran 33 bringbar ist, gebildet.

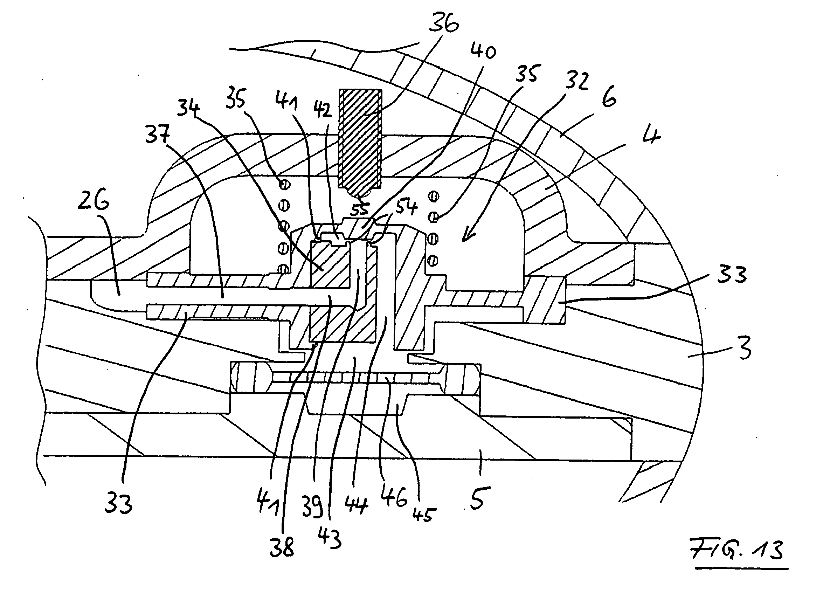

Wie im Detail der Darstellung der Figur 13 zu entnehmen ist, mündet der Kanal 26 in einen radial verlaufenden Kanal 37 der Ventilmembran 33 und von dort in einen radialen Kanal 38 des Ventilkörpers 34, der in einen axialen Kanal 39 des Ventilkerns 34 mündet. Dieser Kanal 39 ist im Bereich seines, einem verstärkten Abschnitt 40 der Ventilmembran 33 zugewandten Endes offen ausgebildet. Auf der dem Kanal 39 abgewandten Seite des Abschnitts 40, dem die Funktion eines Verschlusselements zukommt, ist ein Anschlag, der als Stellschraube 36 ausgebildet ist, angeordnet. Grundsätzlich könnte dieser Anschlag auch stationär sein. Zwischen den Vorsprüngen 41 der Ventilmembran 33 ist der Ventilkern 34 axial unverschieblich bezüglich der Ventilmembran 33 gehalten und im Übrigen auch bezüglich dieser nicht drehbar.

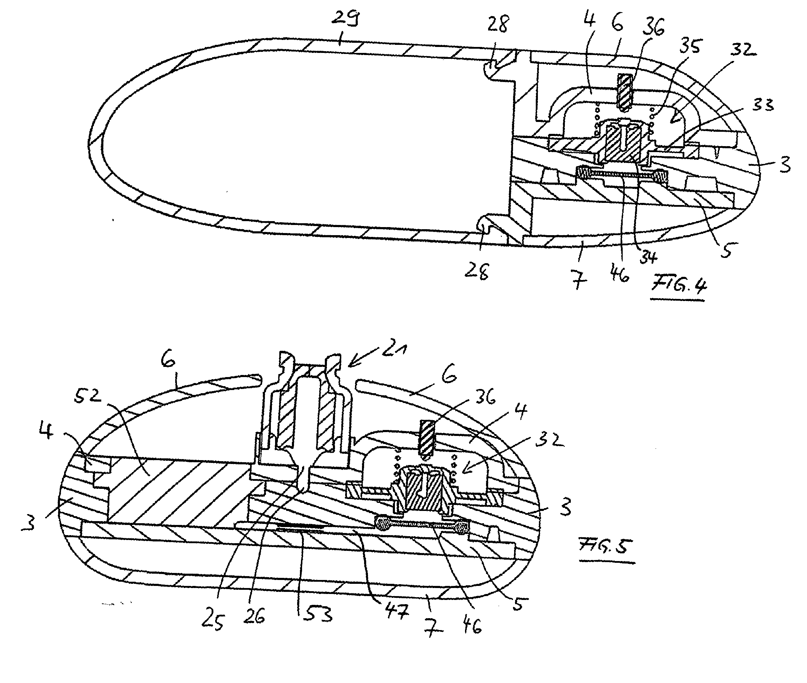

Das Ventil 32 dient dem Sperren des Volumenstromes bei Anliegen eines zu hohen Drucks. Im Ventil sind zwei getrennte Kammern 42 und 43 gebildet, die miteinander über einen den Ventilkern 34 durchsetzenden, parallel zum Kanal 40 angeordneten Kanal 44 verbunden sind. Dabei dient die Kammer 42, welche in Strömungsrichtung zum Zugang, demnach zum Kanal 26 liegt, als Sperrkammer. Die Kammer 43 liegt in Strömungsrichtung zum Abgang 45. Zum Filtern der durch das Ventil 32 ausgegebenen Flüssigkeit ist ein Filter 46 vorgesehen, der randseitig zwischen dem Mittelteil 3 und dem Unterteil 5 geklemmt ist. Ausgehend von der Kammer 43 und dem Abgang 45 gelangt die Flüssigkeit zu einem mit dem Abgang 45 in Fließverbindung stehenden Kanal 47 (Figur 5) und von dort zu einem zwischen dem Mittelteil 3 und dem Unterteil 5 gehaltenen LuerLock-Anschluss 48. Mit diesem ist ein Luer-Lock-Verbinder 49 verbindbar, der mit einem Schlauch 50, der zum Patienten führt, versehen ist.

Wie der Darstellung der Figur 5 zu entnehmen ist, ist in den Kanal 47 eine Glaskapillare 53 eingesetzt. Diese stellt einen Durchflussbegrenzer dar, der in der Lage ist, den Volumenstrom, der durch den Kanal 47 aus der Pumpe austritt, zu begrenzen, weil der Durchflussbegrenzer einen kleineren Querschnitt aufweist, als der im Zugang liegende Kanal 37. Durch die Wahl verschiedener Durchflussbegrenzer ist die Einstellung verschiedener konstanter Flowraten möglich, solange der Eingangs des Eintritts anliegende Druck einen bestimmten Wert nicht unterschreitet. Grundsätzlich ist vorgesehen, dass der Durchflussquerschnitt des Zugangs größer ist als der Durchflussquerschnitt des Abgangs. Selbstverständlich kann der Durchflussbegrenzer anders ausgeführt sein, als in Art einer Glaskapillare. Es ist durchaus denkbar, hinter dem Ventil im Abgang der Pumpe, beispielsweise einen Mäander-Chip, der den Durchfluss begrenzt, vorzusehen.

Auf Grund der angegebenen Durchmesser der Kanäle, die den Raum 27 des Ballons mit dem Ventil 32 verbinden und des Durchmessers der hinter dem Ventil 32 angeordneten Kanäle mit dem Durchflussbegrenzer 53, ist der Widerstand, den der Kanal 47 mit dem Durchflussbegrenzer 53 dem Auströmen der Flüssigkeit aus dem Gehäuse 2 entgegensetzt, größer als der Widerstand, der der Flüssigkeit beim Einströmen in das Ventil 32 entgegen gesetzt wird.

In einer Ausgangssituation befindet sich die Ventilmembran 33 in der in Figur 13 gezeigten Position, in der die Ventilmembran 33, ohne dass es einer Einwirkung der Druckfeder 35 bedürfte, weitgehend am Mittelteil 3 anliegt. Auf Grund der Positionierung des Ventilkerns 34 bezüglich des Abschnitts 40 der Ventilmembran 33 ist ein geringer Spalt zwischen dem Abschnitt 40 und einem umlaufenden, somit ringförmigen Vorsprung 54 des Ventilkerns 34 gegeben. Dieser Vorsprung 54 umschließt den Kanal 43. Demzufolge strömt Flüssigkeit über den Kanal 13 des Kerns 11 und den anschließenden, gehäuseseitigen Kanal 26 in den Kanal 37 der Ventilmembran 33 und von dort in die Kanäle 38 und 39 des Ventilkerns 34. Vom Kanal 39 des Ventilkerns 34 strömt die Flüssigkeit durch den zwischen dem Vorsprung 54 und dem Abschnitt 40 der Ventilmembran 33 gebildeten Spalt in die dort befindliche Kammer 42, und von der Kammer 42 über den Kanal 44 zwischen Ventilmembran 33 und Ventilkern 34 zur Kammer 43, passiert den Filter 46 und gelangt über den Abgang 45 zum Kanal 47 mit dem Durchflussbegrenzer 53. Stellt sich im Einlass, somit auch im Kanal 39, ein höherer Flüssigkeitsdruck ein, ohne dass in Folge des Durchflussbegrenzers 43 ein größerer Volumenstrom aus der Pumpe austreten kann, führt dies dazu, dass die Ventilmembran 33, die in dem Randbereich zwischen dem Mittelteil 3 und dem Oberteil 4 geklemmt ist, sich im zentralen Bereich in Richtung der die Anschlagfunktion aufweisenden Stellschraube 36 verformt, und zwar entgegen der Kraft der Druckfeder 35. Gelangt die Ventilmembran 34 mit ihrem Abschnitt 40 gegen den Vorsprung 55 der Stellschraube 36, der dem Abschnitt 40 zugewandt ist, kontaktiert der Abschnitt 40 dort die Stellschraube 36, womit, da sich die Ventilmembran 33 nicht weiter nach oben in Richtung der Oberschale bewegen kann, der Abschnitt 40 gegen den Vorsprung 54 des Ventilkerns 34 gedrückt wird und damit den Durchfluss durch den Kanal 39 verschließt. Beim Abfließen der Flüssigkeit durch den Durchflussbegrenzer 53 baut sich der Druck in der Kammer 43 ab, sodass die Membran, durchaus auf Grund deren eigenen Elastizität, wieder in Richtung ihrer Ausgangsstellung gemäß Figur 13 zurückbewegt, damit der Abschnitt 40 außer Kontakt mit der Stellschraube 36 gelangt und der Durchflussspalt zwischen dem Vorsprung 54 und dem Abschnitt 40 wieder freigegeben wird. In Abhängigkeit vom Druck, der in dem Ballon 16 herrscht, kann dieser Zustand erst bei Erreichen der Ausgangsposition der Ventilmembran 33, wie sie in Figur 13 dargestellt ist, oder durchaus schon früher, somit bei noch ausgelenkter Ventilmembran 33, eintreten. Die Stellschraube 36 dient der Veränderung des Öffnungs-Schließ-Verhaltens des Ventils 32. Je weiter die Schraube den Stellweg der Ventilmembran freigibt, desto größer ist der Sekundärdruck im Ventil. Grundsätzlich ist es nicht erforderlich, die Druckfeder 35 vorzusehen. Sie ist dann von Vorteil, wenn größere Drücke mit der Pumpe 1 beherrscht werden sollen und demzufolge das elastische Rückstellverhalten der Ventilmembran 33 nicht ausreicht, um sie in die Ausgangsstellung gemäß Figur 13 zu überführen.

Mit dem Ventil 32 wird somit der Flüssigkeits-Volumenstrom in Abhängigkeit vom anstehenden Druck im Ballon 16 begrenzt und über den Durchflussbegrenzer 53 der Flüssigkeits-Volumenstrom weitgehend konstant gehalten. Grundsätzlich könnte die Flüssigkeitspumpe so modifiziert werden, dass nur eine Einrichtung zum weitgehenden Konstanthalten des vom elastischen Element ausgegebenen Flüssigkeits-Volumenstroms oder eine Einrichtung zum Begrenzen des vom elastischen Element ausgegebenen Flüssigkeits-Volumenstroms vorgesehen ist.

Vor dem Benutzen der mechanisch betriebenen Flüssigkeitspumpe wird Flüssigkeit durch das Luer-Lock-Ventil 21 zugeführt, womit die Flüssigkeit in den Ballon 16 gelangt und der Füllstand des Ballons sich durch die durchsichtige Haube 29 an Hand der in Querrichtung der Haube angebrachten Markierungen 51 ablesen lässt, die eine Referenz für die Querausdehnung des Ballons in Abhängigkeit von dessen Befüllung sind. Nach dem Befüllen der Pumpe 1 und dem Anschließen der Pumpe an den Patienten über den Schlauch 50, wird, unter elastischer Vorspannung des aufgeweiteten Ballons 16, Flüssigkeit über das Ventil 32 aus der Pumpe ausgegeben, und zwar solange, bis der Ballon restlos entleert ist und an dem Kern 11 anliegt.

Durch die besonders einfache Gestaltung der beschriebenen Flüssigkeitspumpe bietet diese vielfältige Möglichkeiten der Verwendung bzw. Anwendung. Der Anwender kann überall, sofort, ohne längere Anlaufzeiten die Pumpe einsetzen. Sie kann vom Anwender tragbar, wie auch statisch benutzt werden, und zwar in allen normalen Lebensbereichen außerhalb, wie innerhalb der Medizin. Die Pumpe ist sterilisiert einsetzbar und gewährleistet einen minimalen Bedienungs/Handlingaufwand. Die Pumpe ist auf Grund der einfachen Konstruktion der wenigen Bauteile kostengünstig herstellbar. Dies ist Vorraussetzung dafür, dass sie insbesondere in ambulanten und finanzschwachen Märkten eingesetzt werden kann. Das geringe Gewicht der Pumpe ermöglicht den Einsatz im Unfall-, Notarzt-, Lazarett- sowie Katastrophenbereich. Die Funktionselemente der Pumpe sind einzeln oder insgesamt austauschbar. Die Pumpe ist für kurze oder lange Förderzeiten geeignet, beispielsweise bei einem Ballon mit einem Fassungsvermögen von 25 ml für eine Durchflussrate von 2,5 ml pro Stunde, somit einer Laufzeit von 10 Stunden. Selbstverständlich können andere Ballons Verwendung finden, die andere Volumina aufweisen, z. B. 10 ml, 50 ml, 100 ml oder 150 ml. Die Laufzeit kann durchaus wesentlich länger sein, beispielsweise bis 24 Stunden. Obwohl Flowraten von > 1000 ml pro Stunde durchaus denkbar sind, wird als bevorzugtes Anwendungsgebiet eine Flowrate von 0,5 bis 10 ml pro Stunde angesehen.

Gemäß dem Ausführungsbeispiel wird eine spritzgusstechnisch hergestellter Ballon beschrieben, der als Sammelbehälter für die Medikamentenlösung und Druckreservoir dient. Der Ballon weist eine definierte Kontur im Querschnitt und in der Ausdehnung, zum Füllen flacher Gehäuseräume und zur Vermeidung von Druckspitzen auf. Er steht radial und/oder axial unter Vorspannung über einen ein- oder mehrteiligen Kern, zur Erhöhung der Rückstellkräfte. Der Ballon wird einseitig über den Kern ortsfest mittels eines Klemmrings formschlüssig, luftdicht abgeschlossen. Der Ballon ist frei beweglich in axialer und radialer Richtung während der Befüllung und Entleerung, dabei elastisch verformbar und kann sich reibungsfrei innerhalb der Haube bewegen.

Die Pumpe 1 kann zusätzlich mit einem Bolusreservoir versehen sein. In der Figur 1 ist eine solche Bolus-Vorsehung mit der Bezugsziffer 52 veranschaulicht. Bei Bedarf kann die Pumpe insofern umgerüstet werden.

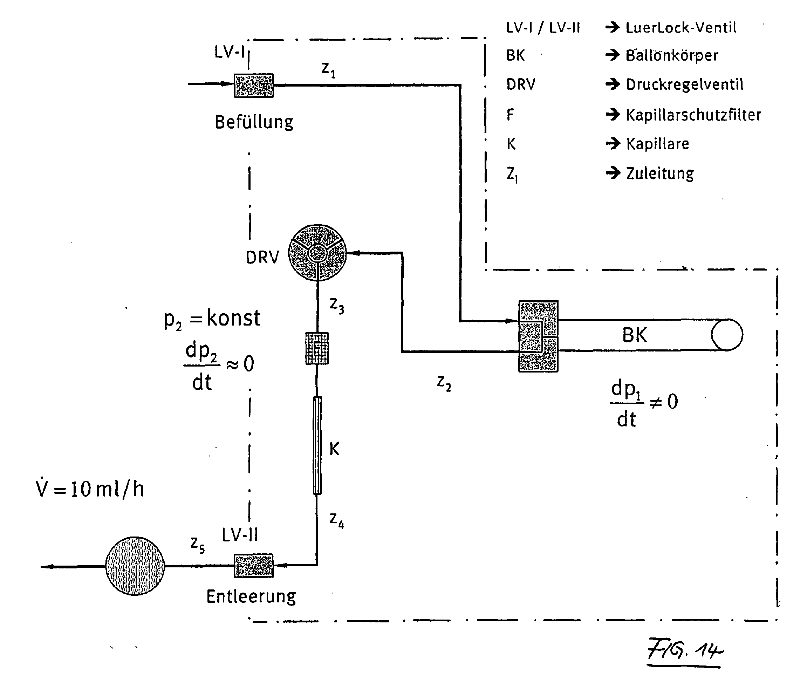

Die Figur 14 veranschaulicht in einem Schaubild das Wirkprinzip der zuvor beschriebenen mechanisch betriebenen Flüssigkeitspumpe mit Angabe der physikalischen Größen. Mit strichpunktierter Linie ist die Kontur der Pumpe verdeutlicht.

## Patentansprüche

1. Ventil (32) für ein Fluid, insbesondere für eine Flüssigkeit, wobei das Ventil (32) durch das zu regelnde Fluid betätigt wird, mit einem Gehäuse (3, 4), wobei das Ventil (32) eine im Gehäuse (3, 4) gelagerte elastische Ventilmembran (33) und einen in dieser gehaltenen Ventilkörper (34) aufweist sowie mit einem Zugang (26, 37, 38, 39) für das Fluid, der in eine Kammer (42, 43, 44) mündet, und einem von der Kammer (42, 43, 44) abgehenden Abgang (45), wobei der Ventilkörper (34), zum Schließen des Fluiddurchgangs des Ventils (32), in Kontakt mit einem elastischen Element (33) bringbar ist, **gekennzeichnet durch** folgende Merkmale:
- das elastische Element (33) ist die elastische Ventilmembran (33),
- ein sich außerhalb des Fluidweges befindlicher Anschlag (36, 55) dient dem Begrenzen der Auswölbbewegung der Ventilmembran (33) bei einem Druckanstieg des Fluids, wobei der Anschlag (36, 55) das elastische Element (33) gegen den Ventilkörper (34) bewegt und den Fluiddurchgang (26, 37, 38, 39, 42, 43, 44, 45) des Ventils (32) schließt,
- der Zugang (36, 37, 38, 39) ist **durch** die Ventilmembran (33) und den Ventilkörper (34) hindurch zur Kammer (42, 43, 44) geführt,
- der am Ventilabgang anstehende Druck führt über eine entsprechend groß gestaltete Wirkfläche zu einer Auswölbung der Ventilfläche.

2. Ventil nach Anspruch 1, **dadurch gekennzeichnet, dass** die Ventilmembran (33) und der Ventilkörper (34) separate Bauteile darstellen, insbesondere der Ventilkörper (34) als starres Bauteil ausgebildet ist.

3. Ventil nach einem der Ansprüche 1 oder 2, **dadurch gekennzeichnet, dass** die Ventilmembran (33) im Bereich eines umlaufenden Randes zwischen zwei Teilen (3, 4) des Gehäuses (3, 4) gehalten, insbesondere klemmend gehalten ist.

4. Ventil nach einem der Ansprüche 1 bis 3, **dadurch gekennzeichnet, dass** die Ventilmembran (33) im Querschnitt profiliert ausgebildet ist und ein zentraler Bereich der Ventilmembran (33) der Aufnahme des als Ventilkern ausgebildeten Ventilkörpers (34) dient.

5. Ventil nach einem der Ansprüche 1 bis 4, **dadurch gekennzeichnet, dass** ein Verschlusselement (40), das Bestandteil der Ventilmembran (33) ist, vorgesehen ist, wobei der Anschlag (36, 55) das Verschlusselement (40) gegen den Ventilkörper (34) bewegt und den Fluidzugang (26, 37, 38, 39) des Ventils (32) schließt.

6. Ventil nach einem der Ansprüche 1 bis 5, **dadurch gekennzeichnet, dass** der Abgang von der Kammer (42, 43, 44) durch die Ventilmembran (33) hindurchgeführt ist.

7. Ventil nach einem der Ansprüche 1 bis 6, **dadurch gekennzeichnet, dass** die Ventilmembran (33) einen radialen Einlasskanal (37) und der Ventilkörper (34) einen sich an diesen Einlasskanal (37) anschließenden radialen Einlasskanal (38) aufweist, wobei letzterer (38) in einen axialen Einlasskanal (39) des Ventilkörpers (34) mündet, der mittels der Ventilmembran (33) verschließbar ist.

8. Ventil nach einem der Ansprüche 1 bis 7, **dadurch gekennzeichnet, dass** ein Teilbereich der Kammer (42, 43, 44) als Kanal (44) ausgebildet ist, der zwischen dem Ventilkörper (34) und der Ventilmembran (33) angeordnet ist.

9. Ventil nach einem der Ansprüche 1 bis 8, **dadurch gekennzeichnet, dass** der Anschlag (36, 55) als verstellbarer Anschlag (36, 55), insbesondere als in das Gehäuse (Gehäuseteil 4) eingeschraubte Steilschraube (36) ausgebildet ist.

10. Ventil nach einem der Ansprüche 1 bis 9, **dadurch gekennzeichnet, dass** eine Feder (35) zum Rückstellen der Ventilmembran (33) in die Offenstellung des Ventils (32) vorgesehen ist.

11. Ventil nach Anspruch 10, **dadurch gekennzeichnet, dass** die Feder (35) als Druckfeder ausgebildet ist, die sich am Gehäuse (Gehäuseteil 4) und der Ventilmembran (33) abstützt, wobei insbesondere die Ventilmembran (33) gegen einen gehäuseseitigen Anschlag (Gehäuseteil 3) rückstellbar ist.

12. Ventil nach einem der Ansprüche 1 bis 11, **dadurch gekennzeichnet, dass** der Abgang (45, 47) des Ventils (32) einen Durchflussbegrenzer (53) aufweist.

## Claims

1. Valve (32) for a fluid, in particular for a liquid, the valve (32) being activated by the fluid to be regulated, with a housing (3, 4), the valve (32) having an elastic valve membrane (33) mounted in the housing (3, 4) and a valve body (34) held in the elastic valve membrane (33), and with an inlet (26, 37, 38, 39) for the fluid, opening into a chamber (42, 43, 44), and with an outlet (45) leading out from the chamber (42, 43, 44), it being possible to bring the valve body (34) into contact with an elastic element (33) in order to close the fluid passage of the valve (32), **characterized by** the following features:
- the elastic element (33) is the elastic valve membrane (33),
- a stop (36, 55), located outside of the fluid path, is used to limit the extent to which the valve membrane (33) curves outward during an increase in pressure of the fluid, the stop (36, 55) moving the elastic element (33) against the valve body (34) and closing the fluid passage (26, 37, 38, 39, 42, 43, 44, 45) of the valve (32),
- the inlet (36, 37, 38, 39) is routed through the valve membrane (33) and the valve body (34) to the chamber (42, 43, 44),
- the pressure arising at the valve outlet causes the valve surface to curve outwards across a suitably large effective area.

2. Valve according to Claim 1, **characterized in that** the valve membrane (33) and the valve body (34) constitute separate structural parts and, in particular, the valve body (34) is designed as a rigid structural part.

3. Valve according to one of Claims 1 or 2, **characterized in that** the valve membrane (33) is held in the area of a circumferential edge between two parts (3, 4) of the housing (3, 4), and is in particular held by clamping.

4. Valve according to one of Claims 1 to 3, **characterized in that** the valve membrane (33) is profiled in cross section, and a central area of the valve membrane (33) serves to receive the valve body (34) designed as valve core.

5. Valve according to one of Claims 1 to 4, **characterized in that** a closure element (40) is provided, which is a component part of the valve membrane (33), and the stop (36, 55) moves the closure element (40) against the valve body (34) and closes the fluid passage (26, 37, 38, 39) of the valve (32).

6. Valve according to one of Claims 1 to 5, **characterized in that** the outlet from the chamber (42, 43, 44) is routed through the valve membrane (33).

7. Valve according to one of Claims 1 to 6, **characterized in that** the valve membrane (33) has a radial inlet channel (37) and the valve body (34) has a radial inlet channel (38) adjoining this inlet channel (37), said channel (38) merging into an axial inlet channel (39) of the valve body (34), which can be closed by means of the valve membrane (33).

8. Valve according to one of Claims 1 to 7, **characterized in that** a partial area of the chamber (42, 43, 44) is designed as a channel (44), which is arranged between the valve body (34) and the valve membrane (33).

9. Valve according to one of Claims 1 to 8, **characterized in that** the stop (36, 55) is designed as an adjustable stop (36, 55), in particular as an adjusting screw (36) that is screwed into the housing (housing part 4).

10. Valve according to one of Claims 1 to 9, **characterized in that** a spring (35) is provided for resetting the valve membrane (33) to the open position of the valve (32).

11. Valve according to Claim 10, **characterized in that** the spring (35) is designed as a compression spring supported on the housing (housing part 4) and on the valve membrane (33), and, in particular, the valve membrane (33) can be reset against a stop on the housing side (housing part 3).

12. Valve according to one of Claims 1 to 11, **characterized in that** the outlet (45, 47) of the valve (32) has a flow restrictor (53).

## Revendications

1. Soupape (32) pour un fluide, en particulier pour un liquide, la soupape (32) étant actionnée par le fluide à réguler, comprenant un boîtier (3, 4), la soupape (32) présentant une membrane de soupape élastique (33) montée dans le boîtier (3, 4) et un corps de soupape (34) fixé dans celle-ci, ainsi qu'un accès (26, 37, 38, 39) pour le fluide, qui débouche dans une chambre (42, 43, 44), et une sortie (45) partant de la chambre (42, 43, 44), le corps de soupape (34), pour fermer le passage de fluide de la soupape (32), pouvant être amené en contact avec un élément élastique (33), **caractérisée par** les caractéristiques suivantes :
- l'élément élastique (33) est la membrane de soupape élastique (33),
- une butée (36, 55) se trouvant en dehors du trajet du fluide sert à limiter le mouvement de bombement de la membrane de soupape (33) en cas d'augmentation de la pression du fluide, la butée (36, 55) déplaçant l'élément élastique (33) contre le corps de soupape (34) et fermant le passage de fluide (26, 37, 38, 39, 42, 43, 44, 45) de la soupape (32),
- l'accès (36, 37, 38, 39) est guidé à travers la membrane de soupape (33) et le corps de soupape (34) jusqu'à la chambre (42, 43, 44),
- la pression régnant à la sortie de la soupape entraîne, par le biais d'une surface active de taille suffisamment grande, à un bombement de la surface de soupape.

2. Soupape selon la revendication 1, **caractérisée en ce que** la membrane de soupape (33) et le corps de soupape (34) constituent des composants séparés, et en particulier **en ce que** le corps de soupape (34) est réalisé sous la forme d'un composant rigide.

3. Soupape selon l'une quelconque des revendications 1 ou 2, **caractérisée en ce que** la membrane de soupape (33) est maintenue, en particulier par serrage, dans la région d'un bord périphérique entre deux parties (3, 4) du boîtier (3, 4).

4. Soupape selon l'une quelconque des revendications 1 à 3, **caractérisée en ce que** la membrane de soupape (33) est réalisée avec un profilage en section transversale, et une région centrale de la membrane de soupape (33) sert à recevoir le corps de soupape (34) réalisé sous forme de noyau de soupape.

5. Soupape selon l'une quelconque des revendications 1 à 4, **caractérisée en ce qu'**il est prévu un élément de fermeture (40) qui fait partie de la membrane de soupape (33), la butée (36, 55) déplaçant l'élément de fermeture (40) contre le corps de soupape (34) et fermant l'accès du fluide (26, 37, 38, 39) de la soupape (32).

6. Soupape selon l'une quelconque des revendications 1 à 5, **caractérisée en ce que** la sortie de la chambre (42, 43, 44) est guidée à travers la membrane de soupape (33).

7. Soupape selon l'une quelconque des revendications 1 à 6, **caractérisée en ce que** la membrane de soupape (33) présente un canal d'entrée radial (37) et le corps de soupape (34) présente un canal d'entrée radial (38) se raccordant à ce canal d'entrée (37), le canal d'entrée (38) débouchant dans un canal d'entrée axial (39) du corps de soupape (34), qui peut être fermé au moyen de la membrane de soupape (33).

8. Soupape selon l'une quelconque des revendications 1 à 7, **caractérisée en ce qu'**une région partielle de la chambre (42, 43, 44) est réalisée sous forme de canal (44), qui est disposé entre le corps de soupape (34) et la membrane de soupape (33).

9. Soupape selon l'une quelconque des revendications 1 à 8, **caractérisée en ce que** la butée (36, 55) est réalisée sous forme de butée réglable (36, 55), en particulier sous forme de vis de réglage (36) vissée dans le boîtier (partie de boîtier 4).

10. Soupape selon l'une quelconque des revendications 1 à 9, **caractérisée en ce qu'**un ressort (35) est prévu pour ramener la membrane de soupape (33) dans la position d'ouverture de la soupape (32).

11. Soupape selon la revendication 10, **caractérisée en ce que** le ressort (35) est réalisé sous la forme d'un ressort de pression, qui s'appuie contre le boîtier (partie de boîtier 4) et la membrane de soupape (33), la membrane de soupape (33) pouvant notamment être ramenée contre une butée du côté du boîtier (partie de boîtier 3).

12. Soupape selon l'une quelconque des revendications 1 à 11, **caractérisée en ce que** la sortie (45, 47) de la soupape (32) présente un limiteur de débit (53).
